# EUROPEAN PATENT APPLICATION

(11) **EP 4 625 421 A1**
(43) Date of publication of application: **01.10.2025**
(21) Application number: 23894719.6
(22) Date of filing: 08.08.2023
(51) Int. Cl.: G16B 40/20, G16B 30/10

(54) **METHOD AND COMPUTER PROGRAM FOR PREDICTING NEOANTIGENS BY PROCESSING LENGTHS OF PEPTIDES OF VARIOUS LENGTHS BY FOLDING**

(30) Priority: 25.11.2022 KR 20220160675
(71) Applicant: Theragen Bio Co., Ltd., Gyeonggi-do 13488 (KR)
(72) Inventor: HONG, Seongeui, Seongnam-si, Gyeonggi-do 13488 (KR); OH, Sejin, Seongnam-si, Gyeonggi-do 13488 (KR); PAIK, Soonmyung, Seongnam-si, Gyeonggi-do 13488 (KR)
(74) Representative: Müller Schupfner & Partner Patent- und Rechtsanwaltspartnerschaft mbB (Muc)
(86) International application number: PCT/KR2023/011701
(87) International publication number: WO 2024/111786

(57) **Abstract**

Disclosed is a method for processing peptide sequences exceeding a unit length through a process of folding when predicting neoantigens using peptide sequences and HLA class I and/or HLA class II allele sequences. According to the method, peptide sequences contained in cancer tissue can determine neoantigens in the cancer tissue, regardless of the diversity in length of the peptide sequences. Accordingly, it is possible to overcome the imbalance and lack of information in training data on length and more accurately predict a binding force to determine the neoantigens in the cancer tissue.

## Description

### Technical Field

Embodiments of the present invention relate to a method and a computer program for predicting a neoantigenic probability of peptides extracted from cancer tissue and cancer-derived DNA with respect to HLA class I and/or II alleles.

### Background Art

Cancer is one of the most common causes of death worldwide. About 10 million new cases occur each year, and account for about 12% of all deaths, making it the third leading cause of death. Recently, due to advances in the development of new anticancer drugs, recent third-generation immunotherapy drugs are receiving attention via first-generation anticancer drugs which are chemotherapy drugs and second-generation targeted anticancer drugs. In particular, the third-generation immunotherapy drugs have the advantage of significantly lower side effects due to a treatment strategy that utilizes the patient's own immune system, unlike previous anticancer drugs. However, despite these advantages, there is a limitation that a treatment strategy using immunotherapy drugs can be established for only patients with expression of marker genes such as PD-L1 and microsatellite instability-high (MSI-H). Due to these limitations, it is necessary to establish a strategy to treat patients who have difficulty receiving conventional anticancer drugs, and one of alternatives is proposed as a cancer vaccine utilizing a neoantigen.

The immune system has learned not to attack normal cells by eliminating self-aware T cells in the thymus (thymic selection), which is called central tolerance. Cancer is a disease that occurs when mutations accumulate in the genome. Solid cancer cells such as stomach cancer, colon cancer, and breast cancer have an average of 60 mutations, while lung cancer and melanoma caused by carcinogens have even more mutations of about 150.

When the mutations occur, the immune system produces abnormal proteins that have been never seen before. In this case, since the immune system has not encountered the mutated protein during the thymic selection, immune-centric tolerance does not act on the mutated protein. Accordingly, all mutant proteins may be considered to potentially have immunogenicity. However, most mutations include intracellular proteins that cannot be in contact with the surface of tumor cells and thus cannot be in direct contact with immune cells. The mutations may be in contact with immune cells only by a method of presenting 8 to 10 short peptide fragments, called epitopes, on the surface of cancer cells through a class 1 major histocompatibility complex (class 1 MHC, also known as Human Leukocyte Antigen (HLA) in humans). At this time, the mutant peptide that binds to class 1 MHC to be presented on the surface of tumor cells is called a neoepitope or neoantigen, and the neoepitope and MHC complex is called pMHC (or pHLA in humans). The neoepitope may be a kind of marker that helps CD8 cytotoxic T cells recognize cancer cells as a foreign attacker.

However, in order for the immune response to the mutations in cancer cells to be activated, a cancer cell-derived neoantigen pMHC needs to be cross-presented to T immune cells by antigen presenting cells (APCs), especially dendritic cells other than cancer cells, along with accessory proteins expressed only on dendritic cells. Unlike other somatic cells, APCs present longer peptides as neoantigens through class II MHC, which activates CD4 T helper cells as accessory cells, and ultimately, class 1 pMHC, class 2 pMHC, and co-stimulatory molecules all act to generate CD8 cytotoxic T cells with strong immunogenicity against neoantigens.

Through this process, activated T immune cells attempt to attack cancer cells, but the cancer cells express a shield called an immune checkpoint, which paralyzes the activated T cells and inhibits the immune response (T cell exhaustion). Most immunotherapeutic agents currently in clinical use are immune checkpoint inhibitors. The immune checkpoint inhibitors are effective only in some cancers that already have a large number of activated and then paralyzed T cells. However, in many cases, a state of immunological ignorance occurs in which T cells are not initially activated against a neoantigen, and an immune response does not occur. Recently, it has been found that a phenomenon occurs in which the immune response is activated even for neoantigens that had immunological ignorance during neoantigen-targeting cancer vaccine treatment. In other words, it is expected that the neoantigen-targeting cancer vaccine can be applied to cancer types that are not applied with conventional immune checkpoint inhibitors, and a greater therapeutic effect may be expected in combined therapy even in a target group of immune checkpoint inhibitors.

Conventional cancer vaccines targeted treatment of 'tumor-associated antigens', which are 'normal' proteins that are abnormally expressed in higher amounts in cancer cells than in normal cells. Off-the-shelf vaccines may be made by using these commonly occurring targets, but have the disadvantage of low antigenicity due to central tolerance, and no vaccine has yet been proven to have a clear clinical effect. Unlike these 'tumor-associated antigens', neoantigens do not respond to central tolerance and thus may be ideal targets for cancer vaccines.

On the other hand, an HLA gene, which encodes an MHC protein, has the highest polymorphisms of 13,000 or more. These polymorphisms cause differences in peptide binding groove sequence of the MHC protein, and thus the MHC protein has various affinities for peptides. Therefore, a peptide with the same mutation may be presented as a neoantigen only in patients with a specific HLA type that has high affinity for some MHC proteins. Due to such MHC restriction, only less than 10% of mutated peptides are presented by MHC, and most neoantigens are expressed differently in each patient, which is why cancer vaccines need to be customized to each patient.

One of many challenges that arise during the development of cancer vaccines arises from various lengths of neoantigens that bind to HLA. These various lengths cause limitations to establishment of AI-based prediction models, including deep learning. To solve these problems, conventional methods have adopted strategies such as developing models for each length independently or developing models after calibrating the model to a specific length. However, this preprocessing process has disadvantages such as insufficient learning data and loss of information. Accordingly, the present invention intended to resolve the diversity of neoantigen sequences while minimizing data shortage and information loss.

### Detailed Description of the Invention

### Technical Problem

The present invention is based on the aforementioned necessity, and an object of the present invention is to predict the immunogenicity of peptides by using a model learned from data in which peptide sequences of different lengths are each folded and unified into a unit length, to predict a binding force, which is a binding force with HLA alleles, and to determine neoantigens in cancer tissue based on the predicted binding force and immunogenicity.

### Technical Solution

According to embodiments of the present invention, a method may include inputting, by a neoantigen prediction device, a peptide sequence extracted from a target cancer tissue and determining whether the length of the peptide sequence exceeds a predetermined unit length; determining the remaining region excluding a predetermined fixed region in the peptide sequence when the peptide sequence exceeds the unit length, and processing the remaining region so that values of a region exceeding the unit length are folded to reduce the peptide sequence to the unit length; inputting, by the neoantigen prediction device, a sequence of a complex of HLA class I or II alleles; extracting, by the neoantigen prediction device, feature values from input data including sequence values included in the peptide sequence processed and outputting immunogenicity prediction value for the feature values; extracting, by the neoantigen prediction device, peptide feature value from data processed to reduce the peptide sequence to a unit length; one-hot processing, by the neoantigen prediction device, an alpha chain sequence or a beta chain sequence of the HLA class I or II alleles, and extracting HLA feature value corresponding to the processed chain sequences; outputting, by the neoantigen prediction device, a combinability prediction value by a combination of the peptide feature value and the HLA feature value; and determining, by the neoantigen prediction device, a neoantigen of the peptide sequence based on the immunogenicity prediction value or the combinability prediction value.

The reducing the peptide to the unit length may be processed by a folding process in which a first value at a first location and a second value at a second location are folded to be expressed in a sum of the first value and the second value in the remaining region of the peptide and the length of the peptide is reduced by 1.

A fixed region of the peptide may include first, second, and last values, and the values included in the fixed region may not be folded.

The folding process may be repeated until the peptide reaches the unit length.

The outputting the combinability prediction value may be performed by outputting a combinability prediction value using a combinability prediction model, and the combinability prediction model may be a model trained by inputting peptide feature values extracted from peptide sequences, HLA-alpha combinability feature values extracted from alpha chain sequences of HLA class I or II alleles, and HLA-beta feature values extracted from beta chain sequences of HLA class II alleles, and outputting a combinability prediction value corresponding to a binding force.

The combinability prediction model may further include a peptide processing model for extracting peptide feature value, an HLA alpha processing model for extracting HLA-alpha feature values, and an HLA beta processing model for extracting HLA-beta feature values, and the peptide, HLA alpha and HLA beta combinability processing models may be different encoders in the form of an encoder, respectively.

The outputting the immunogenicity prediction value may be performed by outputting an immunogenicity prediction value using an immunogenicity prediction model, and the immunogenicity prediction model may be a model trained by inputting feature values extracted from peptide sequences and outputting an immunogenicity prediction value corresponding to the immunogenicity for the peptide sequences.

The target cancer tissue may include cells engineered to express a single HLA class I and/or class II allele.

The target cancer tissue may be obtained from a plurality of organisms or may include mutant cells derived therefrom.

The target cancer tissue may include fresh or frozen tumor cells obtained from the plurality of organisms.

The target cancer tissue may include fresh or frozen tissue cells obtained from the plurality of organisms.

### Effects of the Invention

According to the embodiment of the present invention described above, it is possible to predict immunogenicity of peptides, predict a binding force with HLA alleles, and determine a neoantigen in a cancer tissue based on the predicted binding force and immunogenicity by using a model trained by data in which peptide sequences of different lengths are folded and unified to a unit length, respectively.

### Brief Description of Drawings

FIG. 1 is a block diagram of a neoantigen prediction device 100 according to embodiments of the present invention.
FIG. 2 is an exemplary diagram of a learning model 200 according to embodiments of the present invention.
FIG. 3a is an exemplary diagram of peptide sequences processed by a method according to embodiments of the present invention. FIG. 3b is an exemplary diagram of input data processed by a conventional method.
FIG. 4a is an exemplary diagram of one-hot encoded peptide sequences according to embodiments of the present invention.
FIG. 4b is an exemplary diagram of folded peptide sequences according to embodiments of the present invention.
FIG. 4c is an exemplary diagram of peptide sequences applied with weight values according to embodiments of the present invention.
FIG. 5 is a flowchart of a method for predicting immunogenicity according to embodiments of the present invention.
FIG. 6 is a diagram comparing a neoantigen prediction model according to embodiments of the present invention with a conventional neoantigen prediction model.

### Modes of the Invention

Hereinafter, configurations and operations of the present invention will be described in detail with reference to embodiments of the present invention illustrated in the accompanying drawings.

The present invention may have various modifications and various embodiments, and specific embodiments will be illustrated in the drawings and described in detail in the detailed description. Advantages and features of the present invention, and methods for accomplishing the same will be more clearly understood from embodiments described in detail below with reference to the accompanying drawings. However, the present invention is not limited to embodiments disclosed below but may be implemented in various different forms.

Hereinafter, preferred embodiments of the present invention will be described in detail with reference to the accompanying drawings, in which like reference numerals refer to like or corresponding elements regardless of reference numerals and a duplicated description thereof will be omitted.

In embodiments below, the terms first, second, etc. are not used in a limiting sense, but used for the purpose of distinguishing one component from the other component.

In embodiments below, a singular expression includes a plural expression unless the context clearly indicates otherwise.

In embodiments below, the term "including", "having", or the like means the presence of features or components described in this specification, but does not exclude in advance the possibility that one or more other features or components may be added.

In the drawings, components can be exaggerated or reduced in size for convenience of explanation. For example, the size and thickness of each configuration illustrated in the drawings are arbitrarily shown for convenience of explanation, and thus the present invention is not necessarily limited to those illustrated above.

When any embodiment is otherwise feasible, a specific processing order may also be performed in a different order to be described. For example, two processes to be described sequentially may be performed substantially simultaneously, or may be performed in an opposite order to the order to be described.

Here, target cancer tissue refers to a tissue to be tested. For example, the target cancer tissue may be a cancer tissue to detect an antigen capable of causing an immune response. Preferably, the target cancer tissue may be an aggregate of tumor cells or cancer cells.

Here, mutation refers to all phenomena in which the arrangement of base sequences A (adenine), T (thymine), G (guanine), and C (cytosine) of genes containing genetic information in each organism changes to be different from original genetic information of the corresponding species. These mutations cause small or large structural mutations. Small mutations may include point mutations, which occur when a single base sequence is changed, and mutations in which base sequences are additionally inserted or deleted. Mutations that occur on a large scale and affect the structure include gene duplication, gene deletion, chromosomal inversion, interstitial deletion, chromosomal translocation, loss of heterozygosity, etc.

The mutations are broadly classified into germinal mutations and somatic mutations depending on a type of cell to be generated. Somatic mutations are genetic mutations that occur in somatic cells, and are also called somatic mutations or somatic variations, and may be caused by gene mutations or chromosomal abnormalities.

The occurrence of these mutations may cause changes in the function of proteins produced by the corresponding gene, and a specific function may be lost or also be activated to another function. Since these changes in protein function may cause or accelerate the development of cancer, these mutations may be directly or indirectly deeply related to the development and progression of cancer.

The base sequence in DNA, which contains the genetic information of an organism, consists of A, T, G, and C, and three of the base sequences are combined in a row to become a code that forms a specific amino acid, and several of these codes are combined to be converted into a protein. Amino acids consist of alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), pyrrolysine (Ply), proline (Pro), glutamine (Gln), arginine (Arg), serine (Ser), threonine (Thr), selenocysteine (Sec), valine (Val), tryptophan (Trp), and tyrosine (Tyr).

A peptide may mean a peptide or a polypeptide consisting amino acid sequences. Within organisms, there is an immune system to remove foreign substances that are not derived from genetic information of each species, and in particular, among foreign peptides, there are immunogenic peptides that may induce immune responses. Mutations that occur differently from the original genetic information during the cancer development process also produce immunogenic peptides, and these immunogenic peptides produced in this way can bind to HLA class I or HLA class II protein via a series of processes within the immune system. Furthermore, the immunogenic peptide may have a mutant amino acid sequence, the amino acid length thereof may be 8 to 12 amino acids for a neoantigen that binds to HLA class I, or 13 to 20 amino acids for a neoantigen that binds to HLA class II, but is not limited thereto and may be various lengths.

Here, machine learning refers to optimizing parameters with given data using a model consisting a plurality of parameters. The machine learning may be divided into supervised learning, unsupervised learning, and reinforcement learning depending on a type of learning problem. The supervised learning is a process of learning mapping between inputs and outputs, and may be applied when pairs of inputs and outputs are given as data.

In the present specification, a neoantigen may be a peptide that induces an immune response, i.e., an immunogenic peptide. The neoantigens may be induced by specific mutations in tumor cells and may be expressed as epitopes on tumor cells. Hereinafter, for brevity of description, the immunogenic peptides are referred to as neoantigens.

A neoantigen prediction device according to embodiments of the present invention may analyze a peptide sequence of a target cancer tissue and an HLA class I allele sequence of an organism to determine whether a specific peptide of a target cancer tissue to be used in the treatment of the target cancer tissue may be determined as a neoantigen. The length of the peptide sequence of the target cancer tissue may not be constant.

Here, a human leukocyte antigen (HLA) is a protein present on the surface of human cells, and may have various types of gene sequences for each organism. In addition, the protein sequence of HLA varies for each organism. Based on this, each organism has different antigenicity.

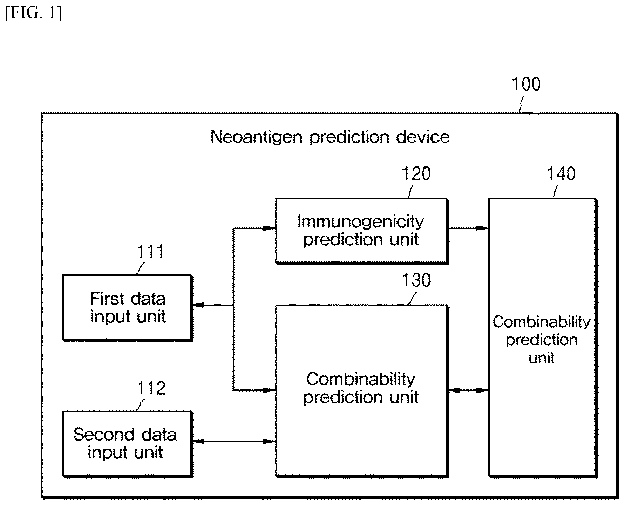

FIG. 1 is a block diagram of a neoantigen prediction device 100 according to embodiments of the present invention.

The neoantigen prediction device 100 may include a first data input unit 111, a second data input unit 112, an immunogenicity prediction unit 120, a combinability prediction unit 130, and a neoantigen prediction unit 140.

The neoantigen prediction device 100 may determine a neoantigen, which is an immunogenic peptide sequence for treating cancer tissues and cancer cells, based on peptide sequences of cancer tissues, cancer cells, etc. and HLA class I and/or II sequences of an organism. At this time, the neoantigen prediction device 100 may determine a neoantigen among the peptide sequences based on the immunogenicity of the peptide sequence and/or the combinability between the peptide sequence and HLA class I and/or II.

The neoantigen prediction device 100 may use a peptide derived from a mutation in a cancer tissue of an organism as a neoantigen and predict a binding force between a mutation-derived peptide and a specific HLA class I and/or II allele of the organism, so that the immune system of the organism may recognize and attack the neoantigen of the peptide.

The neoantigen prediction device 100 may determine whether a mutant-derived peptide having various lengths is a neoantigen by preprocessing the peptide sequence.

The neoantigen prediction device 100 may fold sequence values of a partial region of the peptide sequence to unify the length of the peptide into a predetermined unit length. In the case of the peptide, natural folding may occur, and even in a peptide region, a folding region and a non-folding region may be determined. More specifically, it can be seen that the peptide spontaneously folds in the middle portion. The neoantigen prediction device 100 may fix a region in the peptide sequence where folding occurs with a relatively low probability so as not to be folded. The neoantigen prediction device 100 may determine a fixed region that is not folded in the peptide sequence based on data on the folding each peptide sequence.

The sequence values of the region to be folded in the peptide sequence may be calculated by applying a weight value of a predetermined type. The weight value may be determined based on the number of folding times, the number of values combined by the folding process, etc. For example, if the folding is performed 4 times, a weight value of 4 may be applied to the values of the folded region. When five values are combined and converted into one value, a weight value of 5 may be applied to values of the folded region.

The neoantigen prediction device 100 may predict combinability corresponding to a binding force with a peptide sequence by preprocessing a complex of specific HLA class I and II alleles of an organism. According to an embodiment, the neoantigen prediction device 100 may infer combinability corresponding to the binding force with the peptide sequence after one-hot processing HLA class I and II alleles.

The neoantigen prediction device 100 may calculate an immunogenicity prediction value using an immunogenicity prediction model trained by inputting data obtained by folding the peptide sequence. The neoantigen prediction device 100 may output a combinability prediction value of the peptide sequence using a combinability prediction model trained by inputting data obtained by folding the peptide sequence.

In addition, the neoantigen prediction device 100 may generate a combinability prediction model trained by inputting data encoded with HLA class I and/or II alleles using a method such as one-hot processing. The neoantigen prediction device 100 may input HLA class I and/or II alleles into a combinability prediction model to output a combinability prediction value of HLA class I and/or II alleles. The neoantigen prediction device 100 may output an immunogenicity prediction value using an immunogenicity prediction model, output a combinability prediction value using a combinability prediction model, and output data on a neoantigen using a neoantigen prediction model for the immunogenicity prediction value and the combinability prediction value.

The first data input unit 111 inputs peptide sequences extracted from target cancer tissue or cancer cells. A selected peptide sequence is input from among the plurality of peptides extracted from the target cancer tissue or cancer cells.

The first data input unit 111 may one-hot encode the input peptide sequence and then generate folded data. More specifically, the first data input unit 111 may determine whether the length of the input peptide sequence exceeds a predetermined unit length. The first data input unit 111 may fold a peptide sequence exceeding a unit length and normalize the folded peptide sequence to a unit length.

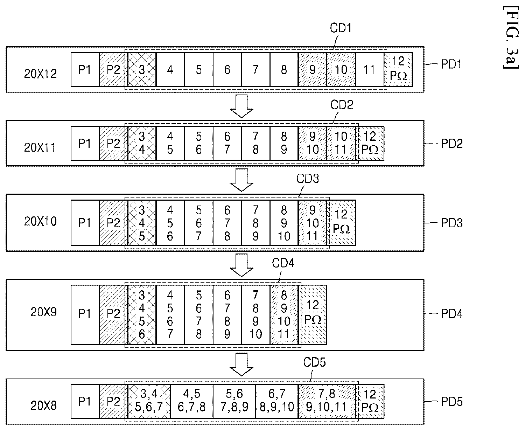

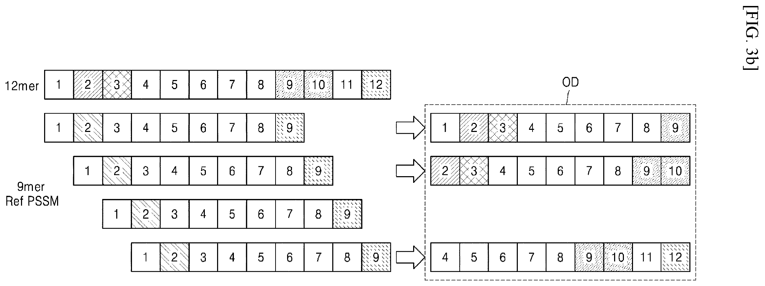

With regard to folding, as illustrated in FIG. 3, the third to eleventh sequence values CD1 excluding P1, P2, and P12 that are fixed in a 12 mer peptide sequence PD1 may be processed into an 11 mer peptide sequence PD2 by folding. In order to reduce the eleventh sequence value in the 12 mer peptide sequence PD1, the third and fourth sequence values of PD1 may be combined and converted into the third sequence value. More specifically, the third and fourth sequence values of PD1 may be combined, or the third or fourth sequence value of PD1 may be converted into the third sequence value. A value obtained by combining the third and fourth sequence values of PD1 may be converted into the third sequence value by applying a predetermined weight value.

The fourth and fifth sequence values of PD1 may be combined to be converted into the fourth sequence value. The fifth and sixth sequence values of PD1 may be combined to be converted into the fifth sequence value. The sixth and seventh sequence values of PD1 may be combined to be converted into the sixth sequence value. The seventh and eighth sequence values of PD1 may be combined to be converted into the seventh sequence value. The eighth and ninth sequence values of PD1 may be combined to be converted into the eighth sequence value. The ninth and tenth sequence values of PD1 may be combined to be converted into the ninth sequence value. The tenth and eleventh sequence values of PD1 may be combined to be converted into the tenth sequence value.

The 11 mer peptide sequence PD2 may be converted into a 10 mer peptide sequence PD3 by folding. The 10 mer peptide sequence PD3 may be converted into a 9 mer peptide sequence PD4 by folding. The 9 mer peptide sequence PD4 may be converted into an 8 mer peptide sequence PD5 by folding.

The second data input unit 112 inputs the sequence of HLA class I and/or II in order to confirm whether the peptide is a neoantigen. The second data input unit 112 may encode the sequences of HLA class I and/or II using a one-hot method, etc.

The immunogenicity prediction unit 120 may output an immunogenicity prediction value of a peptide sequence based on data on the folded peptide sequences.

The immunogenicity prediction unit 120 may obtain immunogenicity values for one or more amino acid sequences included in the folded peptide sequence from an external database. The immunogenicity prediction value of the peptide sequence for a combination of the immunogenicity values may be output to an immunogenicity prediction model trained by machine learning.

The immunogenicity prediction model may be machine-learned using input data for one or more amino acid sequences included in the folded peptide sequence and output data for a value corresponding to the immunogenicity of the peptide sequence. Alternatively, the immunogenicity prediction model may be machine-learned by a combination of immunogenicity values for one or more amino acid sequences included in the folded peptide sequence as input data and a value corresponding to the immunogenicity of the peptide sequence as output data.

The immunogenicity prediction model may be learned specifically by supervised learning, but is not limited thereto and may be learned by unsupervised learning or reinforcement learning. The immunogenicity prediction model may output an immunogenicity prediction value for the peptide sequence by learning input data with one or more neural networks.

The immunogenicity prediction model may be implemented to output an immunogenicity prediction value corresponding to a peptide sequence based on learned data, even when there is no learning data for one or more amino acid sequences included in the folded peptide sequence. The immunogenicity prediction model may infer an immunogenicity prediction value for an unlearned peptide sequence by inputting immunogenicity data for each of amino acid sequences included in the peptide sequence. The immunogenicity prediction model may infer an immunogenicity prediction value for an unlearned peptide sequence based on the immunogenicity value of each of a plurality of amino acid sequences included in the peptide sequence.

More specifically, the immunogenicity prediction model may fold data that has been embedded and encoded to separate the peptide sequence into amino acid sequence units and convert the sequence units into values according to the presence or absence of each amino acid sequence. For example, the peptide sequence may be converted into values (0 or 1) for the first to n-th amino acid sequences. The converted values may be input data for the immunogenicity prediction model.

The immunogenicity prediction model is learned with input data corresponding to folded peptide sequences and output data including immunogenicity values of the peptide sequences, in which the immunogenicity values of the peptide sequences may be determined as immunogenicity values for one or more amino acid sequences included in the peptide sequence. Immunogenicity values for one or more amino acid sequences included in a peptide sequence or immunogenicity values of peptide sequences may be obtained from prestored data.

The immunogenicity values for a novel peptide sequence may be additionally input into the immunogenicity prediction model and used to learn the immunogenicity prediction model. The immunogenicity prediction model may be continuously upgraded by inputting the novel peptide sequence and the immunogenicity values thereof.

he combinability prediction unit 130 may input a peptide sequence obtained from the first data input unit 111 and an HLA class I and/or II allele sequence obtained from the second data input unit 112, and output a combinability prediction value corresponding to a binding force between the peptide sequence and the HLA class I and/or II allele sequence.

The combinability prediction unit 130 may output a combinability prediction value corresponding to a binding force between a folded peptide sequence and a one-hot processed HLA class I and/or II allele sequence.

The combinability prediction model may be used to output the combinability prediction value. The combinability prediction model may use data on folded peptide sequences and data on one-hot processed HLA allele sequences as input data, extract feature values corresponding to the input data, and output combinability prediction value based on the feature values.

The one-hot encoding method may be performed by converting one or more amino acid sequences included in the peptide sequence or the HLA allele sequence into respective corresponding values thereto. Additionally, according to embodiments of the present invention, the peptide sequence may be one-hot encoded and the one-hot encoded peptide sequence may be folded. Experimentally and empirically, values may be pre-defined for all amino acid sequences included in the peptide sequence. For example, a plurality of peptide sequences may be multi-aligned by position, the peptide sequences may be arranged on an x-axis and a y-axis, and positions with high similarity and positions with low similarity may be extracted from the peptide sequences based on the similarity between the peptide sequence on the x-axis and the peptide sequence on the y-axis. At this time, the positions may be determined based on a standard similarity. Additionally, a processing method for unifying the length of the peptide may be performed. An example of such a processing method may be a folding method.

The folding method may mean combining sequence values of a predetermined region in a one-hot processed peptide sequence according to a predetermined rule to reduce the length of a peptide sequence.

The combinability prediction model may input data on peptide sequences processed by a folding method and data on HLA allele sequences to output a combinability prediction value.

The combinability prediction model may extract one or more feature values that can be utilized as input data corresponding to a peptide sequence and an HLA allele sequence, and an output of combinability between the peptide sequence and the HLA allele sequence. The type of feature value to be extracted may be determined as a result of machine learning, with a combination of combinability values for amino acid sequences included in the peptide sequence as input data and a value corresponding to the combinability between the peptide sequence and the HLA allele sequence as output data. The combinability prediction model may extract key feature values by learning input data with one or more neural networks and output combinability prediction value based on the key feature values.

The combinability prediction model may output a combinability prediction value for a novel peptide sequence based on data learned from other peptide sequences, even if there is no learning data for the input peptide sequence.

The combinability prediction model may fold the length of a peptide sequence according to a predetermined rule, and then output a combinability feature value corresponding to the folded peptide sequence. The combinability prediction model may encode and fold a peptide to output a combinability feature value for the peptide sequence.

The combinability prediction model may use values corresponding to encoded HLA class I and/or II allele sequences as input data, and combinability feature values corresponding to the HLA class I and/or II allele sequences as output data.

The combinability prediction unit 130 may input data on a peptide sequence and an HLA class I and/or II allele and output a combinability prediction value corresponding to a binding force between the peptide sequence and the HLA class I and/or II allele.

In an additional embodiment, the combinability prediction unit 130 may output a combinability prediction value using the combinability prediction model. The combinability prediction model is learned by machine learning. As a result of learning, types of the combinability feature value for the peptide sequence and the combinability feature value for the HLA class allele may also be determined. In the combinability prediction model, the combinability prediction value used as output data may be an empirically or experimentally obtained value for the binding force between the peptide sequence and the HLA class I and/or II alleles, and may be a value obtained from an external database.

The neoantigen prediction unit 140 may output neoantigen data including the possibility of the existence of a neoantigen in the peptide sequence, what the neoantigen is, etc., based on the immunogenicity prediction value and/or the combinability prediction value.

The neoantigen prediction unit 140 may output neoantigen data using a neoantigen prediction model trained by inputting the immunogenicity prediction value and the combinability prediction value.

The neoantigen prediction model may be learned by an immunogenicity prediction value and/or a combinability prediction value as input data, and neoantigen data of peptide sequences for HLA class I and/or II alleles as output data, but is not limited thereto, and may be unsupervised learned or reinforcement learned. The neoantigen data may include the presence or absence of a peptide sequence of a neoantigen, a location of a neoantigen, specificity of a neoantigen, possibility of a neoantigen for each location, etc.

The immunogenicity prediction value and the combinability prediction value may include one or more values of 0 or 1, but are not limited thereto and may include variously defined values between 0 and 1.

The neoantigen prediction device 100 according to embodiments of the present invention may output neoantigen data including the presence or absence of a peptide sequence of a neoantigen, a major location in a neoantigen, specificity of a neoantigen, etc., from a peptide sequence derived from a mutation of a target cancer tissue. In particular, the neoantigen prediction device 100 is meaningful in that it determines whether a peptide sequence is a neoantigen for HLA class I and/or II alleles.

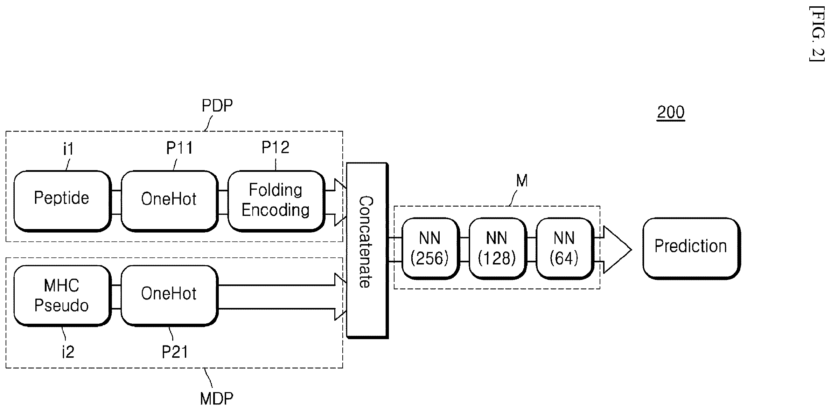

FIG. 2 is an exemplary diagram of the learning model 200 according to embodiments of the present invention.

The learning model 200 may include a neoantigen prediction model M that inputs a peptide sequence i1 and HLA class I and/or II alleles i2 to output immunogenicity data of the peptide.

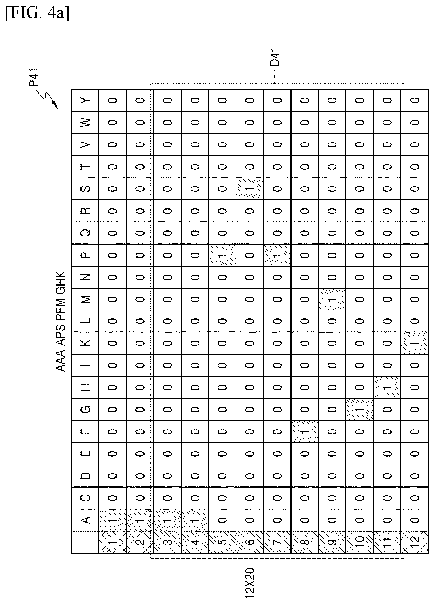

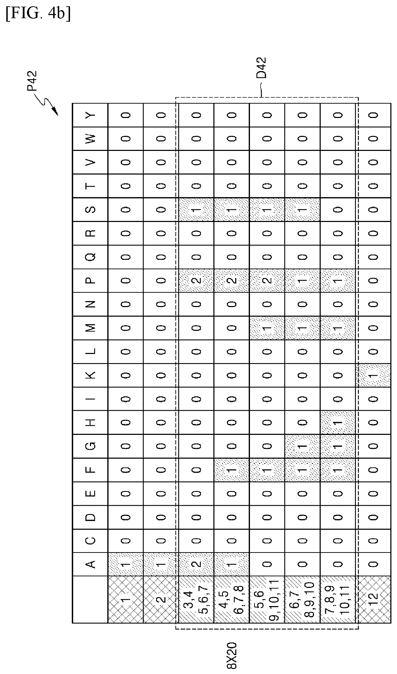

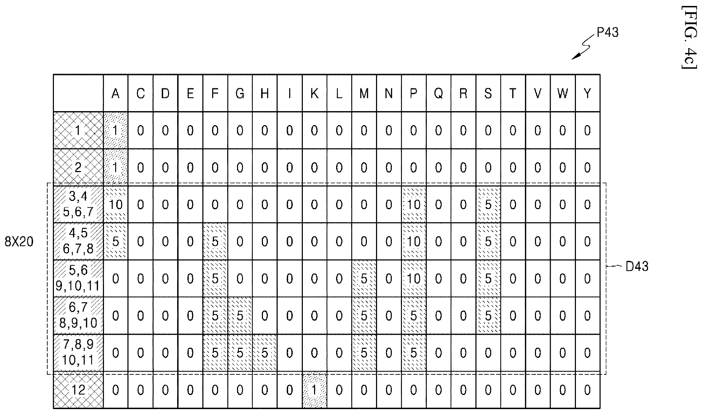

The peptide sequence i1 may be input into the neoantigen prediction model M after a one-hot process P11 and a folding process P12. The one-hot processed peptide sequence is as shown in FIG. 4a. The folding process P12 may convert the combined values into a first value calculated according to a predetermined rule and apply a weight value to the calculated first value to calculate a final second value. The conversion of the combined values into the first value calculated according to a predetermined rule is as shown in FIG. 4b. The predetermined rule may be adding up the combined values, but is not limited thereto, and may be calculated as a weighted average value, an average value, etc. In the learning model 200, two values of the peptide sequence i1 may be folded at a time until becoming a unit length (see FIG. 3a). The final second value is derived by applying a weight value to the first value, as shown in FIG. 4c. The weight value applied at this time may be a value proportional to the number of combined values or the number of folding times, but is not limited thereto and may be determined by combining various values.

The HLA class I and/or II alleles i2 may be input into the neoantigen prediction model M after the one-hot process P21.

The neoantigen prediction model M may input the P11 and P12 processed peptide sequence i1 and the P21 processed HLA class I and/or II allele i2 to output data on the immunogenicity of the peptide sequence i1. At this time, the length of the peptide sequence i1 may be folded into a predetermined unit length, for example, 8 mer. The peptide sequence i1 may undergo a folding process in a unit length for regions excluding the fixed region after the one-hot process. A non-folded fixed region may include first, second, and last values, but is not limited thereto, and may include a critical region. The HLA class I alleles i2 may be one-hot processed based on predetermined amino acid sequences.

The neoantigen prediction model M may be designed by including layers of neural networks NN 256, NN 128, and NN 64. As a result learned by inputting a training data set into the neoantigen prediction model M, the layers of NN 256, NN 128, and NN 64 may be completed. Here, the number of neurons and the structure of layers in NNs are not limited to the examples above and may have different forms.

The neural network may be a set of algorithms that extract various attribute information of input data using a result of statistical machine learning and identify and/or determine objects in an image based on the extracted attribute information. The neural network may be implemented with software or engines, etc. for executing a set of algorithms. The neural network implemented with software or engines, etc. may be executed by a processor in a device or a processor on a server.

The neoantigen prediction model M may include an immunogenicity prediction model that infers the immunogenicity of a peptide sequence and a combinability prediction model. The combinability prediction model may infer combinability between the peptide sequence and the HLA class I and/or II alleles.

The neoantigen prediction model M according to an embodiment may output data on neoantigens as output values, by using a model trained by inputting output values of an immunogenicity prediction model and a combinability prediction model between the HLA class I and/or II and the peptide.

The learning model 200 may be implemented in software or hardware, or implemented as a combination of software and hardware.

The learning model 200 may be provided within the neoantigen prediction device 100 or may be provided in a separate device from the neoantigen prediction device 100. The learning model 200 may be connected via a network or electrically connected with a plurality of neoantigen prediction devices. The learning model 200 may receive data necessary for learning from external devices. The learning model 200 may transmit learned prediction models to neoantigen prediction devices to output neoantigen data for peptide sequences.

FIG. 3a is an exemplary diagram of peptide sequences processed by a method according to an embodiment of the present invention. FIG. 3b is an exemplary diagram of input data processed by a conventional method.

The one-hot processed peptide sequence PD1 has a length exceeding a unit length of 20 × 12 and requires a folding process. The values of P1, P2, and P12, which are fixed regions predetermined in the peptide sequence PD1, may be fixed without folding. The fixed region may be set differently for each peptide sequence. The fixed region is determined based on the probability that folding occurs in each of the peptide sequences, and may be set to a region with a low probability of folding occurring. By fixing some regions and then performing folding, the immunogenicity and combinability of the peptide sequence may be predicted more accurately.

If a region CD1 excluding the fixed region in the peptide sequence PD1 is folded once, the region CD1 is the same as PD2. In the way that the third and fourth values are folded to be calculated into the third value, the third to tenth values CD2 are calculated. The peptide sequence that has been folded once may be changed in length to 20 × 11.

If a region CD2 excluding the fixed region in the peptide sequence PD2 is folded once more, the region CD2 is the same as PD3. In the way that the third and fourth values folded once are folded to be calculated into the third value, the third to ninth values CD3 are calculated. The peptide sequence that has been folded twice may be changed in length to 20 × 10.

If a region CD3 excluding the fixed region in the peptide sequence PD3 is folded once more, the region CD3 is the same as PD4. In the way that the third and fourth values folded twice are folded to be calculated into the third value, the third to eighth values CD4 are calculated. The peptide sequence that has been folded three times may be changed in length to 20 × 9.

If a region CD4 excluding the fixed region in the peptide sequence PD4 is folded once more, the region CD4 is the same as PD5. In the way that the third and fourth values folded three times are folded to be calculated into the third value, the third to seventh values CD5 are calculated. The peptide sequence that has been folded four times may be changed in length to 20 × 8.

In FIG. 3a, the fixed regions were P1, P2, and P12, but in order to increase the probability of predicting immunogenicity, the fixed regions can be changed according to the characteristics of the target cancer, HLA class I, or peptide sequence.

The folding process can also be changed to increase the probability of predicting immunogenicity.

When a method of extracting a sequence that has a maximum value of a diagonal sum after taking the inner product of a PPSM matrix of a conventional peptide sequence and MHC data is applied, there may be a problem of inevitably losing information on one of two anchors. In addition, when using the PSSM matrix, there was a problem that data preprocessing was required manually whenever new reference data was updated. As shown in FIG. 3b, when a method of extracting a sequence that has a maximum value of a diagonal sum after taking the inner product of a PPSM matrix of a peptide sequence and MHC data is applied, it can be seen that the tenth value, the first value, and the first to third values are lost (see OD).

FIG. 4a is an exemplary diagram of one-hot encoded peptide sequences according to embodiments of the present invention.

FIG. 4b is an exemplary diagram of folded peptide sequences according to embodiments of the present invention.

FIG. 4c is an exemplary diagram of peptide sequences applied with weight values according to embodiments of the present invention.

In a peptide sequence P41 of FIG. 4a, P42 of FIG. 4b may be generated by changing the remaining region D41, excluding a predetermined fixed region, to a value D42 combined according to a predetermined rule. At this time, in order to combine a 12 × 20 matrix into an 8 × 20 matrix, five values need to be combined into one value. As illustrated in FIG. 4b, the third sequence value may be determined by combining the third to seventh sequence values according to a predetermined rule. The predetermined rule may be one of a summed value, a weighted average value, and an average value. Optionally, after being combined according to the predetermined rule, P43 may be generated by applying each weight value to each sequence value. As shown in FIG. 4c, processed data P43 and D43 may be generated by applying a multiple of 5 to each of combined values of the peptide sequence. The multiple of 5 is just an example and other multiples may be applied. In FIG. 4a to FIG. 4c, a 12 × 20 peptide sequence was changed to an 8 × 20 peptide sequence with a unit length of 8, but peptide sequences of various sizes of 11 × 20, 10 × 20, 13 × 20, etc. may be changed to a predetermined unit length.

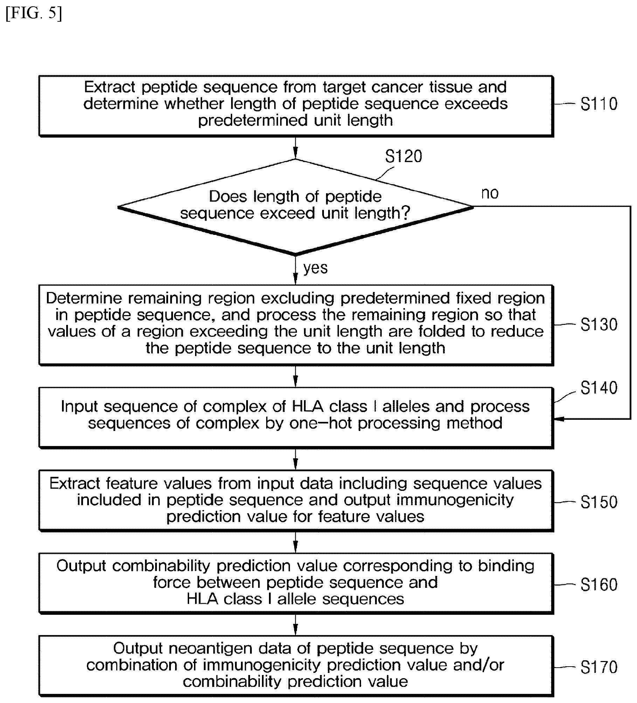

FIG. 5 is a flowchart of a method for predicting immunogenicity according to embodiments of the present invention.

In S110, the neoantigen prediction device 100 inputs a peptide sequence extracted from a target cancer tissue. The neoantigen prediction device 100 may determine whether the length of the peptide sequence exceeds a predetermined unit length.

In S120, the neoantigen prediction device 100 may set the remaining region excluding a predetermined fixed region in a peptide sequence when the length of the peptide sequence exceeds a predetermined unit length, and fold values of a region exceeding the unit length for the remaining region to reduce the peptide sequence to a unit length (S130).

In S140, the neoantigen prediction device 100 may input the sequence of a complex of HLA class I alleles and process the sequences of the complex using a one-hot processing method.

In S150, the neoantigen prediction device 100 may extract feature values from input data including sequence values included in the peptide sequence and output immunogenicity prediction value for the feature values. At this time, the neoantigen prediction device 100 may output immunogenicity prediction value using the learned immunogenicity prediction model.

In S160, the neoantigen prediction device 100 may output a combinability prediction value by one-hot processing data obtained by folding a peptide sequence and HLA class I and/or II allele sequences and inputting the processed sequence into the combinability prediction model.

In S170, the neoantigen prediction device 100 may output neoantigen data of the peptide sequence for the immunogenicity prediction value and/or the combinability prediction value using the immunogenicity prediction model.

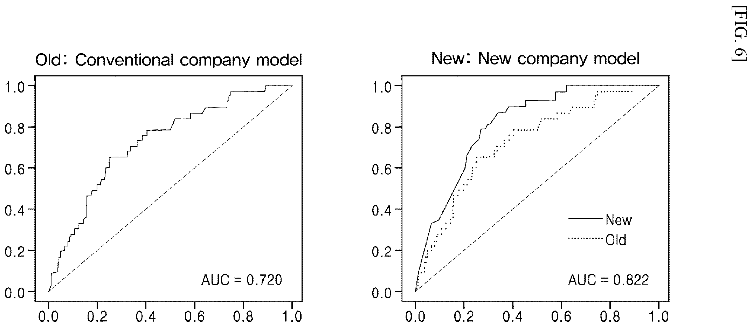

FIG. 6 is a diagram comparing a neoantigen prediction model according to embodiments of the present invention with a conventional neoantigen prediction model.

It was experimentally derived that AUC of the result predicted by inputting the data of the folded peptide sequence was 0.822, which was significantly superior to an AUC value of 0.720 of a result predicted by inputting data of peptide sequences processed by other processing methods.

The neoantigen prediction device 100 may output an immunogenicity prediction value of a peptide sequence from a combination of one or more amino acid sequences included in the folded peptide sequence and immunogenicity values corresponding to one or more amino acid sequences.

The immunogenicity prediction device 100 may obtain immunogenicity values for one or more amino acid sequences included in the peptide sequence from an external database. The immunogenicity prediction value of the peptide sequence for a combination of the immunogenicity values may be output to an immunogenicity prediction model trained by machine learning.

The immunogenicity prediction model may be machine-learned using input data for one or more amino acid sequences included in the peptide sequence and output data for a value corresponding to the immunogenicity of the peptide sequence. Alternatively, the immunogenicity prediction model may be machine-learned by a combination of immunogenicity values for one or more amino acid sequences included in the peptide sequence as input data and a value corresponding to the immunogenicity of the peptide sequence as output data, and may be particularly supervised learned, but is not limited thereto, and may be unsupervised learned or reinforcement learned. The immunogenicity prediction model may extract key feature values by learning input data with one or more neural networks to output an immunogenicity prediction value. The immunogenicity prediction value may include one or more immunogenicity prediction value.

The immunogenicity prediction model may output an immunogenicity prediction value corresponding to a peptide sequence based on learned data, even when there is no learning data for one or more amino acid sequences included in the peptide sequence.

The immunogenicity prediction model may perform an embedding process of separating the peptide sequence into amino acid sequence units and converting the sequence units into values according to the presence or absence of each amino acid sequence. For example, the peptide sequence may be converted into values (0 or 1) for the first to n-th amino acid sequences. The converted values may be input data for the immunogenicity prediction model.

The immunogenicity prediction model is learned with input data corresponding to a plurality of peptide sequences and output data including immunogenicity values of the peptide sequences, in which the immunogenicity values of the peptide sequences may be determined as immunogenicity values for one or more amino acid sequences included in the peptide sequence. The immunogenicity values of the peptide sequences may be obtained from prestored data. The immunogenicity values for a novel peptide sequence may be additionally input into the immunogenicity prediction model and used to learn the immunogenicity prediction model. The immunogenicity prediction model may be upgraded through the novel peptide sequence and the immunogenicity values thereof.

The combinability prediction model may use folded peptide sequences as input data and extract feature values corresponding to the input data. The type of feature value extracted from the combinability prediction model may be determined as a result of machine-learning a combination of combinability values for amino acid sequences included in the peptide sequence as input data and values corresponding to the combinability of the peptide sequence as output data.

The combinability prediction model may input data on peptide sequences processed by a folding method and values corresponding to encoded HLA class I and/or II allele sequences to output a combinability prediction value.

The combinability prediction model may extract key feature values by learning input data with one or more neural networks and output combinability prediction value based on the key feature values.

The combinability prediction model may output a combinability prediction value by outputting a combinability feature value for a peptide sequence based on the learned data, even if there is no learning data for the peptide sequence.

The combinability processing model may fold a peptide sequence to unify the length of the peptide sequence into a unit length, and then calculate amino acid usage features of the binding/non-binding peptide for each length, and extract the corresponding peptide feature value thereto. In another embodiment, the combinability prediction model may convert a peptide sequence into a vector using one-hot encoding, and pass the vector through a plurality of neural networks to output a combinability prediction value.

In an optional embodiment, the combinability prediction model includes one or more prediction models, and each processing model may use, as input data, an alpha chain or a beta chain of an HLA class I allele or II allele, respectively. Each prediction model may output combinability feature values corresponding to HLA class I and/or II alleles. The combinability prediction model may extract key feature values by learning input data with one or more neural networks and output combinability prediction value between peptides corresponding to the key feature values and HLA alleles.

The combinability prediction model may obtain alpha chain sequences of the HLA class I or II alleles extracted from the peptide sequences, and input the alpha chain sequences of the HLA class I or II alleles into an HLA alpha processing model to extract HLA-alpha combinability feature values. The combinability prediction model may extract HLA-beta combinability feature values by inputting the beta chain sequences of the HLA class II alleles into the HLA beta processing model.

The combinability prediction model may be a model trained by inputting peptide feature value extracted from peptide sequences, HLA-alpha combinability feature values extracted from the alpha chain sequences of HLA class I or II alleles, and HLA-beta combinability feature values extracted from the beta chain sequences of HLA class II alleles, and outputting a combinability prediction value corresponding to the binding force.

The combinability prediction model may extract peptide feature value using a peptide processing model from data processed to reduce the peptide sequence to a unit length. The combinability prediction model may one-hot process the alpha chain sequence or the beta chain sequence of the HLA class I or II alleles, extract HLA feature value corresponding to the processed chain sequences, and output a combinability prediction value by a combination of the peptide and the HLA feature values.

The neoantigen prediction device 100 may output data on immunogenicity by combining immunogenicity prediction value and combinability prediction value for peptides and HLA.

The neoantigen prediction device 100 may output neoantigen data including the possibility of the existence of a neoantigen, a location of a neoantigen, the possibility of a neoantigen by location, etc. in a peptide sequence, based on at least one of the immunogenicity prediction value and the combinability prediction value for peptides and HLA.

The neoantigen prediction device 100 may output neoantigen data using a neoantigen prediction model trained by inputting the immunogenicity prediction value and/or the combinability prediction value for peptides and HLA.

The neoantigen prediction model may be learned using the immunogenicity prediction value and/or the combinability prediction value for peptides and HLA as input data, and learned using neoantigen data of peptide sequences for HLA class I and/or II alleles as output data, but is not limited thereto, and may be unsupervised learned or reinforcement learned.

The immunogenicity prediction value and/or the combinability prediction value for peptides and HLA may include one or more values of 0 or 1, but are not limited thereto and may include variously defined values.

The device described above may be implemented in hardware components, software components, and/or a combination of hardware components and software components. For example, the device and the components described in the embodiments may be implemented using, for example, one or more general-purpose computers or special-purpose computers, such as a processor, a controller, an arithmetic logic unit (ALU), a digital signal processor, a microcomputer, a field programmable gate array (FPGA), a programmable logic unit (PLU), a microprocessor, or other any devices capable of executing and responding instructions. The processing device may perform an operating system OS and one or more software applications performed on the operating system. In addition, the processing device may also access, store, manipulate, process, and generate data in response to execution of software. For the convenience of understanding, one processing device may be described to be used, but it can be seen to those skilled in the art that the processing device may include a plurality of processing elements and/or a plurality of types of processing elements. For example, the processing device may include a plurality of processors or one processor and one controller. In addition, other processing configurations, such as a parallel processor are also possible.

Software may include computer programs, codes, instructions, or one or more combinations thereof, and may configure the processing device to operate as desired, or to instruct independently or collectively the processing device. Software and/or data are interpreted by the processing device or may be permanently or temporarily embodied in any type of machines, components, physical devices, virtual equipment, computer storage media or devices, or signal waves to be transmitted, in order to provide instructions or data to the processing device. The software may be distributed on a computer system connected via a network, and may be stored or executed in a distributed method. The software and data may be stored in one or more computer readable recording media.

The method according to the embodiment may be implemented in a form of program instructions which may be performed through various computer means to be recorded in a computer readable medium. The computer readable medium may include program instructions, data files, data structures, and the like alone or in combination. The program instructions recorded in the medium may be specially designed and configured for the embodiments or may be publicly known to and used by those skilled in the computer software art. Examples of the computer readable medium include magnetic media, such as a hard disk, a floppy disk, and a magnetic tape, optical media such as a CD-ROM and a DVD, magnetooptical media such as a floptical disk, and hardware devices such as a ROM, a RAM, and a flash memory, which are specially configured to store and execute the program instructions. Examples of the program instructions include high language codes executable by a computer using an interpreter and the like, as well as machine language codes created by a compiler. The hardware devices may be configured to operate as one or more software modules in order to perform the operations of the embodiments, and vice versa.

As described above, although the embodiments have been described by the limited embodiments and the drawings, various modifications and variations are possible from the above description by those skilled in the art. For example, even if the described techniques are performed in a different order from the described method, and/or components such as a system, a structure, a device, a circuit, and the like described above are coupled or combined in a different form from the described method, or replaced or substituted by other components or equivalents, an appropriate result can be achieved.

Therefore, other implementations, other embodiments, and equivalents to the appended claims fall within the scope of the claims to be described below.

## Claims

1. A method for predicting neoantigens by processing various lengths of peptides by folding, the method comprising:
inputting, by a neoantigen prediction device, a peptide sequence extracted from a target cancer tissue and determining whether the length of the peptide sequence exceeds a predetermined unit length;
determining a remaining region excluding a predetermined fixed region in the peptide sequence when the peptide sequence exceeds the unit length, and processing the remaining region so that values of a region exceeding the unit length are folded to reduce the peptide sequence to the unit length;
inputting, by the neoantigen prediction device, a sequence of a complex of HLA class I or II alleles;
extracting, by the neoantigen prediction device, feature values from input data including sequence values included in the peptide sequence processed and outputting immunogenicity prediction value for the feature values;
extracting, by the neoantigen prediction device, peptide feature value from data processed to reduce the peptide sequence to the unit length;
one-hot processing, by the neoantigen prediction device, an alpha chain sequence or a beta chain sequence of the HLA class I or II alleles and extracting HLA feature value corresponding to the processed chain sequence;
outputting, by the neoantigen prediction device, a combinability prediction value by a combination of the peptide feature value and the HLA feature value; and
determining, by the neoantigen prediction device, a neoantigen of the peptide sequence based on the immunogenicity prediction value or the combinability prediction value.

2. The method of claim 1, wherein the reducingthe peptide to the unit length is processed by a folding process in which a first value at a first location and a second value at a second location are folded to be expressed in a sum of the first value and the second value in the remaining region of the peptide and the length of the peptide is reduced by 1.

3. The method of claim 1, wherein a fixed region of the peptide includes first, second, and last values, and
the values included in the fixed region are not folded.

4. The method of claim 2, wherein the folding process is repeated until the peptide reaches the unit length.

5. The method of claim 1, wherein the outputting the combinability prediction value is performed by outputting the combinability prediction value using a combinability prediction model, and
the combinability prediction model is a model trained by inputting the peptide feature values extracted from the peptide sequences, HLA-alpha feature values extracted from the alpha chain sequences of the HLA class I or II alleles, and HLA-beta feature values extracted from the beta chain sequences of the HLA class II alleles, and outputting the combinability prediction value corresponding to a binding force.

6. The method of claim 5, wherein the combinability prediction model further includes a peptide processing model for extracting the peptide feature value, an HLA alpha processing model for extracting the HLA-alpha feature values, and an HLA beta processing model for extracting the HLA-beta feature values, and
the peptide, HLA alpha and HLA beta combinability processing models are different encoders in a form of an encoder, respectively.

7. The method of claim 1, wherein the outputting the immunogenicity prediction value is performed by outputting the immunogenicity prediction value using an immunogenicity prediction model, and
the immunogenicity prediction model is a model trained by inputting the feature values extracted from the peptide sequences and outputting the immunogenicity prediction value corresponding to the immunogenicity for the peptide sequences.

8. The method of claim 1, wherein the target cancer tissue includes cells engineered to express a single HLA class I or class II allele.

9. The method of claim 1, wherein the target cancer tissue is obtained from a plurality of organisms or includes mutant cells derived therefrom.

10. The method of claim 1, wherein the target cancer tissue includes fresh or frozen tumor cells obtained from a plurality of organisms.

11. The method of claim 1, wherein the target cancer tissue includes fresh or frozen tissue cells obtained from a plurality of organisms.

12. A computer program stored in a computer readable recording medium for executing the method according to any one of claims 1 to 11 using a computer.
